# EUROPEAN PATENT APPLICATION

(11) **EP 2 228 078 A1**
(43) Date of publication of application: **15.09.2010**
(21) Application number: 09425096.6
(22) Date of filing: 09.03.2009
(51) Int. Cl.: A61L 15/26, A61L 15/28, A61L 15/42

(54) **Chitosan and polyurethane foam based medication, production process thereof and use thereof in the wound management**

(71) Applicant: Pietrasanta Pharma S.p.A., 55049 Viareggio (LU) (IT)
(72) Inventor: Neri, Giampaolo, 54100 Ronchi (Massa) (IT)
(74) Representative: Bottero, Carlo

(57) **Abstract**

The present invention relates to a wound medication including a first layer of expanded polyurethane and a second layer, intended for coming into contact with the wound, including chitosan and, optionally, an additional substance capable of promoting the healing of the wound selected from clorexidine, metal silver, iodine-povidone, silver sulfadiazine, calcium alginate, sodium alginate and their mixtures.

The medication of the invention is used for protecting wounds, in particular strongly exuding wounds, such as decubitus ulcers, leg ulcers, first- or second-degree burns, and promoting the cicatrisation thereof.

## Description

The present invention relates to a wound medication, in particular for strongly exuding wounds.

Up to the sixties, skin wounds and lesions were dressed in a dry environment, because it was considered more suitable to avoid bacterial contamination phenomena, also supposing that in the absence of tissue humidity the healing of the wound was promoted.

This concept, however, has been overcame and today it is universally accepted that repair processes of the injured skin tissue are promoted by the humid environment, both with respect to the reepithelialization stimulation and to the cell migration of the neurohumors involved in the physiological functions which lead to the healing of the damaged tissue (Okan D. et al.; The role of moisture balance in wound healing; ADV Skin Wound Care, 2007, 20(1), 55-55).

Therefore, in the last years, medications made of materials capable of promoting and maintaining a humid environment have been developed.

Among the known materials, the expanded polyurethane, permeable to the air and the water vapor, has physical-chemical properties capable of creating an ideal microclimate suitable for accelerating the healing of wounds, decubitus ulcers, leg ulcers, first- and second-degree burns and, generally, strongly exuding wounds.

Sometimes, expanded polyurethanes can be associated with bacteriostatic, haemostatic, anti-inflammatory substances which gain an important role depending on the type of wound to be treated and/or the healing step of the wound itself. Such substances are, for example, biopolymers, such as collagen, gelatin, alginic acid, chitin, chitosan, fibrin, dextran, etc.

The Italian patent application number FI2007A000014 dated January, 26th 2007, describes a wound medication including a layer of expanded polyurethane including medical substances for the protection and the treatment of wounds, particularly chitosan based medical preparations.

EP 0371736 discloses a wound medication including a polyurethane resin film obtainable by the reaction of an isocyanate with a tetrahydrofuran and ethylene oxide random copolymer associated with a sheet made of a biopolymeric material. This latter can be collagen, gelatin, alginic acid, chitin, chitosan, fibrin, dextran, etc. In particular, the employed biopolymer is chitosan or collagen. The collagen preferably has a deacetylation degree equal to 40% or 50% or higher. The polyurethane employed in the medication of EP 0371736 is subjected to boring so as to create holes of proper dimensions through which the exudate produced by the wound is drained.

The inventors of the present patent application have found an alternative solution to the use of a compact polyurethane film subjected to manual or mechanical boring. Such solution relates to the use of an expanded polyurethane.

It has been checked that expanded polyurethane promotes the creation of an ideal microclimate for the healing of the wounds by draining the exudate of the wound and absorbing and holding the humidity in the micro-holes formed during the expansion.

The humidity hold in the micro-holes of the expanded polyurethane promotes and accelerates the healing of the wound, especially of strongly exuding wounds.

The chitosan, another fundamental component of the medication under discussion, is a biopolymer with a high molecular weight formed by glucosamine monomers and obtained with a deacetylation process of the chitin contained in the crustacea exoskeleton. Several studies attribute to the chitosan haemostatic, antibacterial, anti-inflammatory and cicatrizant properties.

The object at the base of the present invention is to provide a wound medication which promotes the cicatrization of lesions with the following mechanisms ensured by the action synergy of the two fundamental constituents (expanded polyurethane and chitosan):
a) Absorption of the wound exudate;
b) Mechanical protection of the wound from external factors;
c) Preservation of the humid environment recognized as optimal for the cicatrization;
d) Release to the wound of a substance having a haemostatic, antibacterial, anti-inflammatory and cicatrizant activity.

A medication thus formed has the advantage that it can be effectively used on each kind of wound and, above all, in each step of the wound, from the bleeding step up to the proliferation and remodeling one.

The wound medication as designed is defined in the appended claims.

The wound medication of the invention includes a first layer of polyurethane and a second layer, intended for coming into contact with the wound, including a biologically active substance and applied on the first polyurethane layer.

The biologically active substance advantageously used for the purposes of the invention is the chitosan, in particular chitosan having a deacetylation degree between 60% and 99%, preferably between 70% and 99%.

The layer including the biologically active substance can further include a polymeric binder, for example selected from polyvinyl alcohol, polyacrylates, polymethacrylates and polyacrylic acid.

In another embodiment, the second layer can include, besides the chitosan, at least an additional substance capable of promoting the healing of the wound. Such substance can have cicatrizant or antiseptic properties and is preferably selected from clorexidine, metal silver, iodine-povidone, silver sulfadiazine, calcium alginate, sodium alginate and their mixtures. This substance can be in a free form or conjugated to the chitosan.

A further object of the present invention is a process for the preparation of the expanded polyurethane/chitosan bilayer, wherein a chitosan-including aqueous based or organic solvent based formulation is spread or nebulized on the expanded polyurethane layer.

The medication of the invention is used for protecting wounds, in particular strongly exuding wounds, such as decubitus ulcers, leg ulcers, first- or second-degree burns and promoting the cicatrisation thereof.

Further features and advantages of the invention will result more apparent by the detailed following description, also carried out with reference to the following figures, in which:
Figure 1 shows a perspective view of a medication according to the present invention;
Figure 2 shows a perspective view of an alternative embodiment of the medication according to the present invention;
Figure 2a shows an enlargement of the detail of the medication of Figure 2 shown by the dotted line;
Figure 3 shows a sectional view of the medication of figure 2;

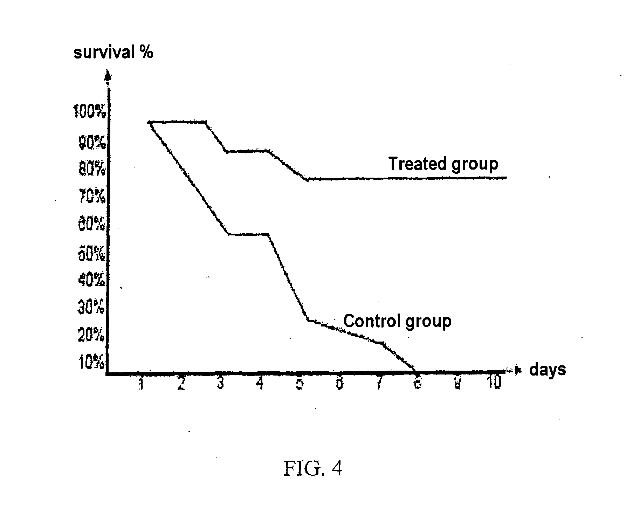

Figure 4 shows a graph representing the percentage of animals survived after 10 days of application of the medication of the invention (treated group) compared to animals treated with a medication including a single layer of polyurethane (control group).

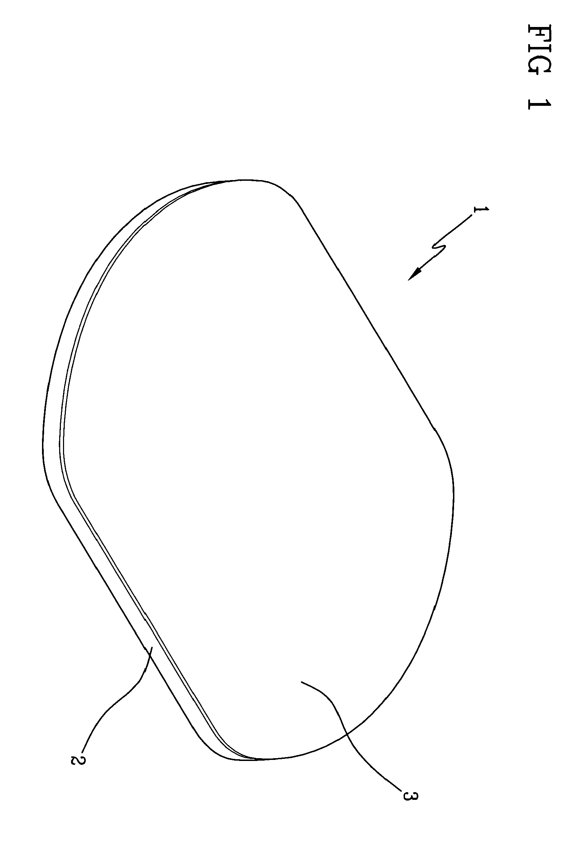

With reference to figures 1, 2, 2a and 3, the medication of the invention, generally shown by numeral 1, includes a first layer of expanded polyurethane 2 and a second layer 3, comprising a biologically active substance, in particular chitosan, applied on the expanded polyurethane layer and intended for coming into contact with the wound.

The second layer 3 is applied on the first layer 2 by means of spreading or nebulization.

The biologically active substance, in particular the chitosan, is included in the second layer in a quantity from 5 to 40 g/m², preferably from 10 to 20 g/m².

The chitosan employed in the present invention can be powdered or fiber chitosan, preferably powdered chitosan.

The second layer, in addition to the chitosan, can include at least an additional substance capable of promoting the healing of the wound.

Such substance can have cicatrizing or antiseptic properties and is preferably selected from clorexedine, metal silver, iodine-povidone, silver sulfadiazine, calcium alginate, sodium alginate and their mixtures.

The second layer can also include a polymeric binder which can be a hydrophilic polymer, for example a polyvinyl alcohol (PVA), or a polyacrylate or a polymethacrylate, for example the Plastoid B, or a polyacrylic acid.

The polymeric binder promotes the adhesion between the polyurethane layer and the chitosan layer and is selected as a function of the preparation process of the expanded polyurethane/chitosan bilayer, as it will be explained hereinbelow in the patent application.

The polymeric binder is included in the second layer in a quantity from 0.5 to 10 g/m², preferably from 2 to 4 g/m² The second layer can also include a plasticizer, such as for example a polyhydroxylated compound, such as glycerin, sorbitol, polyethylene glycol, or triethyl citrate, which helps to increase the flexibility of the medication and therefore to facilitate the application thereof and the contact with the wound.

The plasticizer is included in the second layer in a quantity between 0.2 and 10 g/m², preferably between 1 and 2 g/m².

The second layer further includes at least an organic or inorganic acid, preferably selected among lactic acid, glutamic acid, acetic acid and hydrochloric acid in a quantity between 5 and 40 g/m², preferably between 10 and 20 g/m². The function of the organic or inorganic acid is to protonate the amino groups of the chitosan and therefore to allow the solubilization thereof in an aqueous environment.

The second layer including the chitosan can be formulated as a hydrogel applied on the expanded polyurethane layer by spreading.

Alternatively, the second layer can be formulated as a chitosan dispersion in an organic solvent applied to the expanded polyurethane layer, for example by nebulization or spreading, and following removal of the organic solvent by heating, as it will be explained in detail hereinbelow with reference to the preparation process of the expanded polyurethane/chitosan bilayer. The medication 1 shown in Figure 1 is used for protecting and promoting the cicatrisation both of surface and cavitary wounds.

In another embodiment, the medication can be formed with an adhesivized layer consisting of thin nonwoven fabric (TNT), polyethylene or polyurethane.

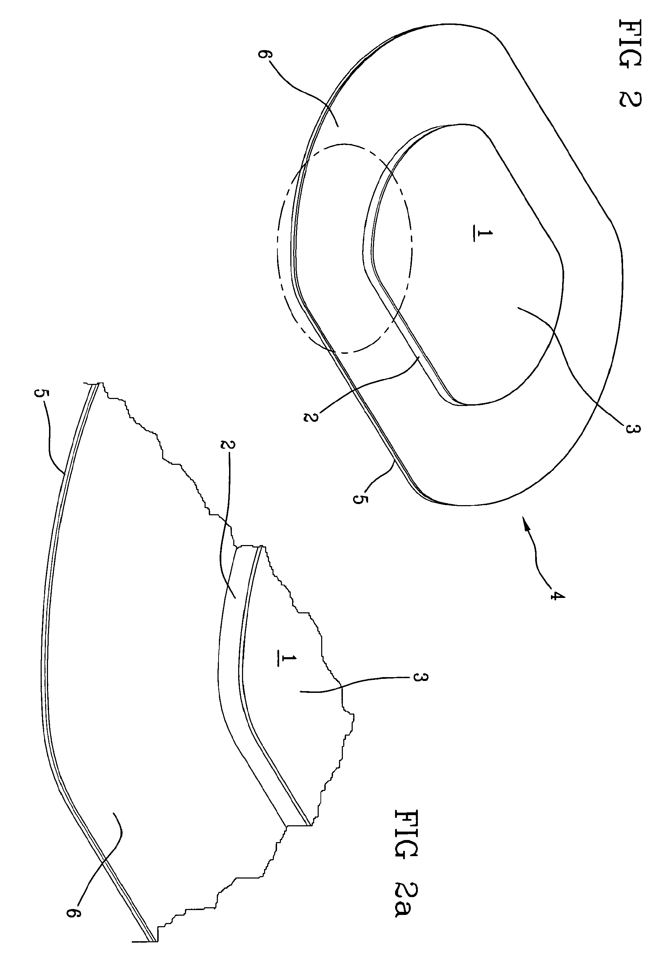

An example of a medication with an adhesivized layer is reported in figures 2-3.

With reference to figures 2-3, number 4 shows the general medication.

The medication 4 includes a layer of nonwoven fabric, polyethylene or polyurethane 5 to which an adhesive layer 6, arranged on the layer 5, is applied.

The adhesive layer 6 allows the fixing of the expanded polyurethane/chitosan bilayer 2, 3 substantially in the proximity of the central portion of the layer 5.

The expanded polyurethane/chitosan bilayer is sized to the layer 5 through the first layer of expanded polyurethane 2 while the second layer 3 including the chitosan is intended for coming into contact with the wound when the medication is applied on the skin.

The application of the medication 4 to the wound is carried out by the portion of layer 5 exceeding the area contacting the expanded polyurethane/chitosan bilayer which, completely or partly covered with the adhesive 6, allows the fixing of the medication to the skin such that the chitosan including-layer contacts the wound.

The expanded polyurethane/chitosan bilayer of the invention can be prepared starting from a formulation containing aqueous based or organic solvent based chitosan.

Such formulation is deposited on an expanded polyurethane layer preferably by spreading or nebulization.

For the preparation of the bilayer, the use of an expanded polyurethane is preferred.

The chitosan including-layer is prepared starting from a composition including chitosan, at least a plasticizer and at least a solvent. Optionally, such composition further includes a substance having a cicatrizing or antiseptic activity as above described.

Such composition is deposited, preferably by spreading or nebulization, on a expanded polyurethane layer; the solvent is then removed.

The used solvent can be water, or a mixture of water and alcohol, or an organic solvent, preferably ethyl acetate.

In particular, the chitosan including-layer can be prepared starting from an aqueous formulation containing chitosan and at least a plasticizer selected from glycerin or other polyalcohols, sorbitol and polyethylene glycols.

Such formulation can also include at least an organic or inorganic acid, preferably selected form lactic acid, glutamic acid, acetic acid and hydrochloric acid and, optionally, a polymeric binder selected from polyvinyl alcohol (PVA), polyacrylic acids and methacrylate polymers.

In this latter case, the polymeric binder is dissolved in water at a temperature from 40°C to 70°C, preferably from 50° to 65°C, in a quantity between 1 and 20% by weight, preferably between 3 and 15% by weight.

After cooling, chitosan, in a quantity from 0.5 to 60% by weight, preferably from 1 to 20% by weight, at least an organic or inorganic acid, preferably selected from lactic acid, glutamic acid, acetic acid and hydrochloric acid in a quantity from 0.5% to 20% by weight, preferably from 2% to 10% by weight, and a plasticizer, in a quantity between 0.5% and 10% by weight, preferably between 2% and 7% by weight, are added, taking care not to incorporate air and slowly mixing until a hydrogel is formed.

In a preferred embodiment, the organic or inorganic acid is employed in the same concentration of the chitosan.

For the preparation of the bilayer, a rolling machine capable of spreading semisolids, such as a hydrogel, on neutral supports, such as the expanded polyurethanes, is used.

A cylinder sliding on the expanded polyurethane allows to deposit the hydrogel on the surface of the foam itself.

The expanded polyurethane/chitosan gel bilayer thus formed is transported in a drying tunnel with a controlled temperature and a forced ventilation, capable of removing the water until to a residual humidity lower than 5%.

After drying, the band is coupled with a film which allows to protect the hydrogel layer from the humidity loss.

Then, the cut of the bilayer is carried out according to the desired dimensions.

All these operations can be automatically carried out with a possibility of changing both the thickness of the chitosan gel and the parameters of the temperature and the ventilation as a function of the preparation that one desires to obtain.

For example, the slip velocity of the cylinder during the spreading can be varied from 0.2 to 15 meters/minute, while the temperature of the drying tunnel can reach up to 150°C.

Temperatures preferably used are between 100 and 120°C.

In an alternative embodiment, a mixture of water and alcohol, preferably ethanol, is used as a solvent.

The water and the alcohol are preferably mixed in a quantity 1:1.

The chitosan is dispersed in a water and alcohol mixture to which a plasticizer, possibly a polymeric binder, and therefore at least an organic or inorganic acid, preferably selected from lactic acid, glutamic acid, acetic acid and hydrochloric acid, are subsequently added under a slow stirring in order to prevent the incorporation of air until the hydrogel is formed.

The gel thus formed is allowed to stand for about 15-20 minutes before the use in order to ensure a complete solubilization of the polymer.

Once the hydrogel has been obtained, the bilayer is prepared as above described with the only exception of the drying temperature that, in this case, is between 75° and 100°C.

In addition to the spreading process of a hydrogel on the expanded polyurethane previously described, an alternative preparation method of the bilayer includes the dispersion of the chitosan (previously treated with the organic or inorganic acid) in an organic solvent and the deposition of the obtained suspension on the expanded polyurethane.

The starting formulation is a suspension in an organic solvent, preferably ethyl acetate, including chitosan in a quantity from 0.5 to 60% by weight, preferably from 1 to 20% by weight, a plasticizer, preferably triethyl citrate, in a quantity between 0.5 and 10% by weight, preferably between 2% and 7% by weight, a polymeric binder, preferably a methacrylic polymer, in a quantity between 1 and 20% by weight, preferably between 3 and 15% by weight.

The deposition of the suspension on the expanded polyurethane layer can take place by nebulization or by spreading.

For the nebulization, the suspension is introduced within a nebulization pump and sprayed on the foam surface through a nozzle with a diameter of about 0.5 mm. The foam thus processed is then dried by evaporation of the solvent with hot air, for example through a passage in a tunnel heated from 70°C to 90°C and with forced ventilation.

For the spreading, the deposition on the expanded polyurethane layer is carried out by the rolling machine already described for the spreading of the chitosan hydrogel; also in this case the removal of the solvent takes place by the passage in a heated and with forced ventilation tunnel.

The bilayer obtained with the methods above described forms the medication subject of the patent, the medication thus obtained can be used as such or can be applied to adhesivized supports as exemplified in Figure 2.

### EXPERIMENTAL PART

### Example 1. Aqueous based formulation

The starting formulation for the preparation of the chitosan hydrogel has the composition reported in the Table 1.

**Table 1**

| Ingredients | Percentage by weight |
|---|---|
| Powdered chitosan | 1-5% |
| 85% lactic acid | 1-5% |
| Polyvinyl alcohol (PVA) | 3-15% |
| Glycerin | 0.5-10% |
| Purified water | q.s. to 100% |

For the preparation of the gel, the PVA was dissolved in water at the temperature of 60°C. After cooling, chitosan, lactic acid and glycerin are added in the order, taking care not to incorporate air, by slowly stirring until the hydrogel is obtained.

For the preparation of the expanded polyurethane/chitosan bilayer, a rolling machine capable of spreading semisolids on neutral supports, as the expanded polyurethanes, was used.

A cylinder which slides on the expanded polyurethane allows to deposit the gel on the surface of the foam itself.

The bilayer thus formed (polyurethane + chitosan gel) is transported in a drying tunnel with controlled temperature and forced ventilation, capable of removing the water up to a residual humidity lower than 5%.

After the drying, the band is coupled with a film which allows to protect from drying the part of foam on which the gel is placed.

Then, the cut of the band is carried out according to the desired dimensions.

### Example 2 - Aqueous based formulation

The starting formulation for the preparation of the chitosan hydrogel has the composition reported in the Table 2.

**Table 2**

| Ingredients | Percentage by weight |
|---|---|
| Powder chitosan | 6.0 |
| 80% lactic acid | 6.0 |
| Glycerin | 10.0 |
| Ethylic alcohol | 39.0 |
| Purified water | 39.0 |

The ethylic alcohol has the function of facilitating the drying after the spreading.

The chitosan is accurately dispersed in the ethanol/water mixture (50:50) to which the glycerin is then added.

Therefore, the lactic acid is added under a slow stirring in order to prevent the air incorporation. The expanded polyurethane/chitosan bilayer was then carried out following the procedure reported in the example 1.

### Example 3. Organic solvent based formulation

The starting formulation to be used is reported in Table 3.

**Table 3**

| Ingredients | Percentage by weight |
|---|---|
| Chitosan | 10-60% |
| Plastoid B | 1-10% |
| Triethyl citrate | 0.5-10% |
| Ethyl acetate | q.s. to 100 |

The chitosan is dispersed in ethyl acetate containing plastoid B and triethyl citrate. Mixing is carried out until a suspension is obtained.

The deposition of the suspension on the foam can take place by nebulization or by spreading.

For the nebulization, the suspension is introduced within a nebulization pump and sprayed on the foam surface by a nozzle with a diameter of about 0.5 mm. The foam thus processed is then dried through evaporation of the solvent with hot air (passage in a tunnel heated at 80°C and with forced ventilation).

For the spreading, the deposition of the suspension on the expanded polyurethane layer is carried out by the rolling machine already described for the spreading of the chitosan hydrogel; also in this case the removal of the solvent takes place by the passage in a heated tunnel and with a forced ventilation.

### IN VITRO TESTS ON THE END PRODUCT

On the end product, obtained according to what reported in the examples 1-2, the following technological tests were carried out:

### 1) absorbing capacity of the liquids

Tests were carried out on the polyurethane as such and the chitosan gel-layered polyurethane. Various samples were contacted with filter paper continuously soaked with water.

At determined times, various samples were weighed in order to evaluate their weight variation as a function of the contacting time with the filter paper.

No statistically significant weight percentage variation was detected between the two series of samples made of polyurethane alone and polyurethane + chitosan gel throughout the duration of the experiment up to 24 hours of contact.

Results obtained with the absorbing capacity test show that the expanded polyurethane maintains the characteristic absorbing capacity of the liquids also in the presence of the chitosan surface layer.

### 2) Penetration of the chitosan within the expanded polyurethane

The chitosan was conjugated with fluorescein isothiocyanate for the purpose of targeting the possible passage of the product within the expanded polyurethane.

The chitosan conjugated with fluorescein isothiocyanate is well visible with naked eye and is orange colored.

Such compound contacted with the wet filter paper does not penetrate within the foam but only passes in solution on the filter paper.

In this way, it is demonstrated that the chitosan exclusively remains into contact with the wound without penetrating the thickness of the foam in the absorbing step of the liquids.

### MOUSE IN VIVO TESTS

A test for evaluating the effectiveness of the preparation of the invention compared with the foam consisting of polyurethane alone was carried out in vivo in a mouse.

Animals used for this study were Swiss mice with a mean weight of 25 grams divided in two groups of 10 animals each.

The test was carried out according to the methodology reported in literature (Burkatvskayia et al. Biomaterials 7 (2006) 4157-4164).

As an effectiveness parameter, the survival percentage of the animals of the two groups at 10 days from the treatment was evaluated (Group no. 1: control treated with expanded polyurethane; Group no. 2: treated with expanded polyurethane + chitosan gel).

As for the preparation of the animals, the dimensions of the wounds reported on the mouse skin and the infection mode, we refer to the methodology of the literature above shown.

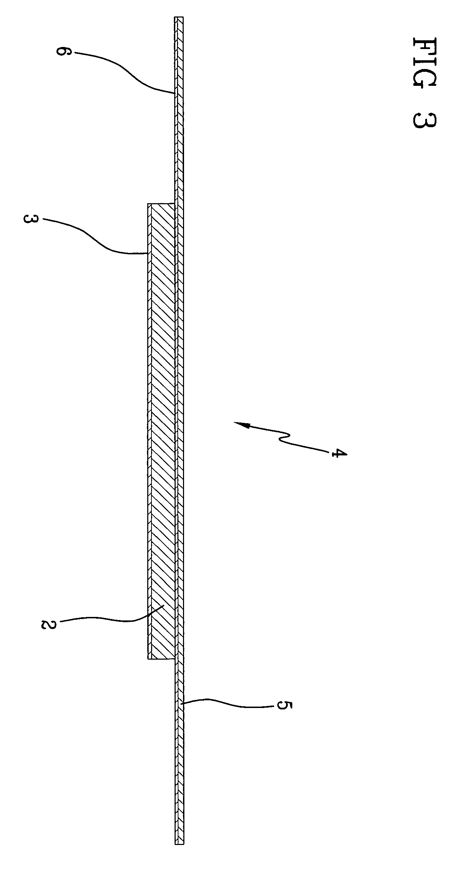

Figure 3 shows the percentage of survived animals (Control group and Treated group) during 10 days of observation.

As it can be seen from figure 3, the group of animals treated with expanded polyurethane + chitosan gel has a survival significantly higher compared with the group of animals treated with polyurethane alone.

Survived animals of the treated group were then controlled for longer times so as to point out the correct cicatrisation of the wounds, which resulted optimal in all the controlled animals. It has been verified that the wound treated with the expanded polyurethane/chitosan bilayer of the invention heals in shorter times than wounds treated with polyurethane in which chitosan is not incorporated.

The experimental results obtained show that:
1) The medication subject of the patent application is able to exert a cicatrizing action in shorter times than a medication formed by expanded polyurethane alone;
2) The production process described is suitable to obtain a preparation which contains, in the outer step, the homogeneously distributed chitosan.

## Claims

1. A wound medication (1), comprising a first layer of expanded polyurethane (2), and a second layer (3) comprising chitosan, intended for coming into contact with the wound, applied on said first layer of expanded polyurethane.

2. A medication according to claim 1, wherein said chitosan is included in said second layer in a quantity from 5 to 40 g/m², preferably from 10 to 20 g/m².

3. A medication according to claim 1 or 2, wherein said chitosan is powdered or fiber chitosan, preferably powdered chitosan.

4. A medication according to any one of the claims 1 to 3, wherein said second layer includes at least an additional substance capable of promoting the healing of the wound.

5. A medication according to claim 4, wherein said at least one additional substance is a cicatrizing or antiseptic substance, in a free form or conjugated to the chitosan, selected form the group consisting of clorexidine, metal silver, iodine-povidone, silver sulfadiazine, calcium alginate, sodium alginate and their mixtures.

6. A medication according to any one of the claims 1 to 5, wherein said second layer further includes a polymeric binder selected from polyvinyl alcohol (PVA), methacrylate polymer and polyacrylic acids.

7. A medication according to claim 6, wherein said polymeric binder is included in said second layer in a quantity from 0.5 to 10 g/m², preferably from 2 to 4 g/m².

8. A medication according to any one of the claims 1 to 7, wherein said second layer further includes a plasticizer selected from glycerin, polyalcohols, triethyl citrate, sorbitol and polyethylene glycols.

9. A medication according to claim 8, wherein said plasticizer is included in said second layer in a quantity from 0.2 to 10 g/m², preferably from 1 to 2 g/m²_{.}

10. A medication according to any one of the claims 1 to 9, wherein said second layer further includes at least an organic or inorganic acid, preferably selected from lactic acid, glutamic acid, acetic acid and hydrochloric acid.

11. A medication according to claim 10, wherein said at least one organic or inorganic acid is included in said second layer in a quantity from 5 to 40 g/m², preferably from 10 to 20 g/m².

12. A medication according to claim 1, wherein said second layer including chitosan is formulated as an hydrogel.

13. A medication according to any one of the claims 1 to 12, further including a nonwoven fabric or polyethylene or polyurethane layer (5) to which an adhesive layer (6) is applied, arranged on said layer (5), which allows the fixing of the expanded polyurethane/chitosan bilayer (2, 3) substantially in the proximity of the central portion of the layer (5).

14. A medication according to any one of the claims 1 to 13 for use in the treatment of surface or cavitary wounds in order to promote the cicatrization thereof.

15. A medication according to claim 14, wherein said wounds are strongly exuding wounds, preferably decubitus ulcers, leg ulcers, first- and second-degree burns.

16. A process for the preparation of the medication (1) according to claim 1 including the steps of:
a) preparing a composition including chitosan, at least a plasticizer and at least a solvent and, optionally, an additional substance capable of promoting the healing of the wound;
b) depositing a layer of said composition on a layer of expanded polyurethane;
c) removing the solvent.

17. A process according to claim 16, wherein the composition of the step a) further includes a polymeric binder.

18. A process according to claim 16 or 17, wherein the composition of the step a) further includes at least an organic or inorganic acid, preferably selected from lactic acid, glutamic acid, acetic acid and hydrochloric acid.

19. A process according to any one of the claims 16 to 18, wherein said solvent is water, or an organic solvent, preferably ethyl acetate, or a water/ - alcohol mixture.
